(19) Europäisches Patentamt
European Patent Office
Office européen des brevets

(11) Numéro de publication: **0 197 869**
**B1**

# (12) FASCICULE DE BREVET EUROPÉEN

(45) Date de publication du fascicule du brevet: **31.05.89**

(21) Numéro de dépôt: **86420093.6**

(22) Date de dépôt: **27.03.86**

(51) Int. Cl.⁴: **C 07 C 43/225**, C 07 C 79/35, C 07 C 49/84, C 07 C 69/92, C 07 C 121/75, C 07 C 149/34, C 07 C 41/16, C 07 C 41/22

## (54) Procédé de préparation de dérivés pentafluoroéthoxy et pentafluoroéthylthiobenzéniques.

(30) Priorité: **29.03.85 FR 8504755**

(43) Date de publication de la demande: **15.10.86 Bulletin 86/42**

(45) Mention de la délivrance du brevet: **31.05.89 Bulletin 89/22**

(84) Etats contractants désignés: **AT BE CH DE FR GB IT LI LU NL SE**

(56) Documents cité:
**EP-A-0 168 344**
**CH-A-525 201**
**DE-B-1 183 096**
**FR-A-2 529 543**
**GB-A-2 045 760**
**US-A-3 207 792**

**THE JOURNAL OF ORGANIC CHEMISTRY, vol. 44, no. 16, 3 août 1979, pages 2907-2910, American Chemical Society; A.E. FEIRING: "Chemistry in hydrogen Fluoride. 7. A novel synthesis of aryl trifluoromethyl ethers"**
**BULLETIN OF THE CHEMICAL SOCIETY OF JAPAN, vol. 50, no. 11, novembre 1977, pages 3069-3070; TAKESHI NAKAI et al.: "A convenient preparation of arylthioynamines"**

(73) Titulaire: **RHONE- POULENC CHIMIE, 25, quai Paul Doumer, F-92408 Courbevoie Cédex (FR)**

(72) Inventeur: **Desbois, Michel, 526, Chemin du Bois, F-69140 Rillieux- la- Pape (FR)**
Inventeur: **Langlois, Bernard, 91, rue Duguesclin, F-69006 Lyon (FR)**

(74) Mandataire: **Le Pennec, Magali, RHONE- POULENC INTERSERVICES Service Brevets Chimie 25, Quai Paul Doumer, F-92408 Courbevoie Cédex (FR)**

**EP 0 197 869 B1**

Il est rappelé que: Dans un délai de neuf mois à compter de la date de publication de la mention de la délivrance du brevet europeen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

**Description**

La présente invention concerne un procédé de préparation de dérivés (pentafluoroéthoxy) et pentafluoroethylthio) benzéniques. Elle concerne plus particulièrement un procédé de préparation desdits composés à partir soit de dérivés phénoliques ou thiophénoliques, soit d'halogénobenzènes.

Il est connu, d'après W.A. SHEPPARD (J.Org.Chemi., 1964, 29, 1), de préparer les (pentafluoroéthoxy) benzènes par fluoration, au moyen du tétrafluorure de soufre, des trifluoroacétates de phényle. Cette méthode n'est pas industrielle car le tétrafluorure de soufre n'est pas disponible industriellement le tétrafluoroacétate de phényle n'est pas lui non plus disponible industriellement. Cette méthode n'est donc pas exploitable.

Il est également connu de préparer les (pentafluoroéthylthio)benzènes par action des thiophénols sur l'iodure de pentafluoroéthyle, en présence d'ammoniac liquide et de radiations ultraviolettes (V.I. Popov et coll. Zh.Org.Khim; 1977, 13 (10), 2135), ou des thiophénates sur les sels d'aryl(pentafluoroéthyl)iodonium (L.M. Yagupolskii et coll., Zh.Org.Khim; 1980, 16 (1), 232). Ces deux méthodes nécessitent d'utiliser l'iodure de pentafluoroéthyle, onéreux et peu disponible industriellement. L'emploi d'ammoniac liquide nécessite également une technologie particulière.

La présente invention a permis d'éviter ces inconvénients.

Il est décrit dans le brevet GB-2 045 760 un procédé de préparation de trifluoroéthoxybenzène par condensation d'un phénol avec le trifluorométhane sulfinate de trifluoroéthyle dans l'acétone.

Il est encore décrit dans le Bulletin de la Société Chimique Japonaise, vol. 50, p. 3069 - 3070 (1977) un procédé de préparation de trifluoroéthylthiobenzène par condensation d'un thiophénate et de tosylane, de trifluoroéthyle en présence d'hydrure de sodium dans le diméthylformamide.

Il est aussi décrit dans le brevet DE-1 183 096 un procédé de chloration de trifluoroéthoxybenzène par addition de trichlorure de phosphore et de chlore sous irradiation.

Le brevet EP-168 344 déposé antérieurement à la présente demande mais publié postérieurement décrit la fluoration des dérivés notamment trifluoro-2,2,2, -dichloro-1,1 éthoxy benzène à l'aide d'un mélange catalytique constitué de pentachlorure d'antimoine et d'acide fluorhydrique. La fluoration échange de groupes trichlorométhylthio fixé sur un noyau benzénique par de l'acide fluorhydrique, du trichlorure d'antimoine ou un mélange des deux.

Le document US-A-3 207 792 décrit un procédé qui consiste à faire un échange chlore-fluor des atomes de chlore portés par un radical méthylthio. Pour réaliser cet échange on peut utiliser l'acide fluorhydrique, $SbF_3$ ou le couple HF et $SbF_3$.

La présente invention décrit un procédé de synthèse de pentafluoroéthoxy ou éthylthiobenzènes à partir d'halogénobenzène ou de phénol.

Elle ne peut être déduite de l'ensemble de ces documents qu'à postériori, l'homme de l'art ne pouvait, avant la lecture du présent texte, réunir ces cinq documents portant sur chacune des étapes intermédiaires.

Elle concerne un procédé de préparation de dérivés (pentafluoroéthoxy) et (pentafluoroéthylthio) benzéniques caractérisé en ce que:

- dans une première étape, on fait réagir:
  . soit un halogénobenzène avec du trifluoroéthanol dans un solvant aprotique en présence d'une base forte et en présence éventuellement d'un sel de cuivre et/ou d'un agent complexant;
  . soit un dérivé phénolique ou thiophénolique avec un dérivé de formule générale (I)

$$CF_3\text{-}CH_2\text{-}O\text{-}R' \qquad (I)$$

dans laquelle R' représente un substituant choisi parmi le groupe comprenant le trifluoracétyle, le méthane sulfonyle, le paratoluène sulfonyle, le trichlorométhane sulfonyle, le chlorosulfonyle, le trifluorométhane sulfonyle en présence d'une base alcaline forte, d'un solvant aprotique et éventuellement d'un agent complexant,

- dans une deuxième étape, on procède à la chloration du dérivé issu de la première étape au moyen de chlore gazeux en présence de radiations et/ou de trichlorure de phosphore,
- dans une troisième étape, on procède à la fluoration du produit issu de la deuxième étape dans l'acide fluorhydrique liquide anhydre en présence d'un acide de Lewis.

Selon le premier procédé de mise en oeuvre de la première étape, c'est-à-dire lorsque celle-ci est effectuée par réaction d'un halogénobenzène et de trifluoroéthanolate dans un solvant aprotique, on fait réagir selon un premier mode préférentiel de mise en oeuvre, un bromo ou un iodobenzène avec le trifluoroéthanolate, en présence de bromure ou de chlorure de cuivre dans un solvant aprotique dipolaire comme le diméthylformamide ou la N-méthylpyrrolidone. Cette réaction est réalisée de préférence à une température comprise entre 80 et 200°C.

Selon un deuxième mode préférentiel de mise en oeuvre du premier procédé de la première étape, celle-ci est effectuée par mise en contact d'un halogénobenzène activé par un substituant électroattracteur choisi, par exemple, parmi les radicaux nitro, cyano, ester, cétone, $CF_3$, avec du trifluoroéthanol, en présence d'une base dans un solvant aprotique.

Cette dernière réaction peut, en outre, être effectuée en présence d'un agent complexant et de préférence en présence d'un agent complexant répondant à la formule générale (II)

$N-[-CHR_1-CHR_2-O-(CHR_3-CHR_4-O-)_n R_5]_3$ (II)

dans laquelle n est un nombre entier supérieur ou égal à 0 et inférieur ou égal à 10 ($0 \leqslant n \leqslant 10$), $R_1$, $R_2$, $R_3$ et $R_4$ identiques ou différents, représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical de formule:

$-C_mH_{2m}C_6H_5$ ou $C_mH_{2m+1}C_6H_4-$,

m étant compris entre 1 et 12.

On utilise encore plus préférentiellement les agents complexants de formule (II) dans laquelle $R_1$, $R_2$, $R_3$ et $R_4$ représente un atome d'hydrogène ou un radical méthyle, $R_5$ représente un groupe méthyle et n est égal à 1. Un solvant aprotique peu ou pas polaire peut alors être employé. Il est de préférence choisi parmi le tétrachlorure de carbone et les chlorobenzènes.

Selon le premier procédé de mise en oeuvre de la première étape de l'invention, c'est-à-dire la mise en contact d'un halogénobenzène et de trifluoroéthanol, on utilise notamment un rapport molaire du trifluoroéthanol au composé halogéné compris entre 0,5 et 5; du trifluoroéthanol à la base, compris entre 1 et 3; et éventuellement du sel de cuivre au composé halogéné compris entre 0,01 et 0,2.

Lorsque l'agent complexant de formule (II) est utilisé, le rapport molaire du dérivé de formule (II) à l'halogénobenzène est compris de préférence entre 0,01 et 0,2.

Selon le deuxième procédé de mise en oeuvre de la première étape, lorsque celle-ci est effectuée par réaction d'un phénol ou d'un thiophénol avec un composé de formule (I), on préfère utiliser comme composé de formule (I) le para toluène sulfonate ou le trichlorométhanesulfonate de trifluoro-2,2,2 éthyle.

Cette réaction peut en outre être effectuée en présence d'un agent complexant et de préférence avec un agent complexant répondant à la formule (II).

Quand on utilise cet agent complexant, la réaction est effectuée de préférence en présence d'une base choisie parmi les hydroxydes, les carbonates de métaux alcalins ou alcalino terreux, le sodium et en présence d'un tiers solvant qui doit solubiliser l'agent complexant et être inerte chimiquement vis-à-vis des composés à dissoudre. Il peut être choisi parmi les solvants aprotiques apolaires ou peu polaires comme, par exemple: le chlorobenzène, l'orthodichlorobenzène, le trichloro-1,2,4 benzène, le tétrachlorure de carbone. On utilise de préférence le trichlorobenzène.

Selon le deuxième procédé de mise en oeuvre de la première étape de l'invention, c'est-à-dire par la mise en contact d'un phénol avec un dérivé de formule (I), on utilise notamment un rapport molaire du phénol ou du thiophénol au composé de formule (I) compris entre 0,5 et 5 et de préférence entre 0,7 et 1,5 et un rapport molaire de la base au phénol ou au thiophénol compris entre 0,5 et 1,1.

Lorsque l'agent complexant de formule (II) est utilisé, le rapport molaire du dérivé de formule (II) au phénol ou au thiophénol est compris de préférence entre 0,01 et 0,2.

La deuxième étape est réalisée notamment par mise en contact de dérivés issus de la première étape avec du chlore gazeux en présence de rayonnements dont les longueurs d'onde sont comprises de préférence entre 250 et 600 nm et en présence éventuellement d'un solvant.

Les solvants utilisés sont des solvants résistant à la chloration tels que le tétrachlorure de carbone et les chlorobenzènes liquides à température ambiante et pression atmosphérique et de préférence le tétrachlorure de carbone. Quand le produit issu de la première étape est liquide et qu'il porte sur le noyau benzénique, un groupe électroattracteur choisi, par exemple, mais non limitativement, parmi les halogènes, les groupes trifluorométhyle, trifluorométhoxy, trifluorométhylthio, nitro, cyano, chlorocarbonyle, etc... la réaction est effectuée avantageusement sans solvant.

La réaction a lieu de préférence à la pression atmosphérique mais dans le cas des composés dont le noyau porte un substituant électroattracteur, une pression supérieure est avantageuse. La température de la réaction est comprise de préférence entre 70 et 200°C et tout particulièrement entre 70 et 85°C lorsque l'on opère en présence de tétrachlorure de carbone.

Les rayonnements sont obtenus généralement par utilisation d'un tube à décharge contenant des gaz rares et/ou de la vapeur de mercure.

La deuxième étape peut aussi être réalisée selon l'enseignement du brevet allemand N° 1 183 096 à l'aide de chlore gazeux en présence de trichlorure de phosphore et de rayonnements et à une température comprise entre 100 et 200°C.

On peut également réaliser cette deuxième étape à l'aide de chlore gazeux en utilisant uniquement le trichlorure de phosphore comme catalyseur et à une température comprise entre 120 et 200°C.

On préfère cependant utiliser le procédé mettant en oeuvre le chlore gazeux en présence de rayonnements.

La troisième étape est effectuée par mise en contact du produit issu de la deuxième étape avec de l'acide fluorhydrique liquide anhydre en présence d'un acide de Lewis.

On préfère utiliser comme acide de Lewis, le pentachlorure d'antimoine ou le trifluorure de bore. Le rapport molaire de l'acide de Lewis au composé issu de l'étape 2 est de préférence compris entre 1 et 20 et encore plus préférentiellement entre 5 et 15 %.

L'acide fluorhydrique est utilisé notamment selon un rapport molaire au composé issu de la deuxième étape

compris entre 5 et 50 et de préférence entre 10 et 30.

La température de la réaction est comprise avantageusement entre 0 et 180°C et encore plus avantageusement entre 80 et 150°C.

Les dérivés (pentafluoroéthoxy) ou (pentafluoroéthylthio) benzéniques sont utilisés comme intermédiaires de synthèse dans l'industrie pharmaceutique, vétérinaire, phytosanitaire et dans les lubrifiants (US-4 366 168, EP-44 808, US-3 265 741).

La présente invention sera plus complètement décrite à l'aide des exemples suivants qui ne doivent pas être considérés comme limitatifs de l'invention.

**Exemple 1**

Synthèse du chloro-4 (trifluoro-2,2,2 éthoxy)benzène à partir de chloro-4 bromobenzène et de trifluoroéthanol.

Dans un ballon de 1 litre à quatre tubulures équipé d'une agitation mécanique centrale, d'une gaine thermométrique et d'un réfrigérant ascendant, on charge 200 ml de diméthylformamide anhydre (fraichement distillé) et 135 g (1,35 mole) de trifluoroéthanol anhydre. On refroidit ce mélange à l'aide d'un bain d'eau glacée puis on rajoute, en 1 heure par petites portions, 65 g d'hydrure de sodium à 50 en suspension dans l'huile de vaseline (soit un total de 32,5 g (1,35 mole) de NaH pur. La température est maintenue, durant cette addition, entre 15 et 17°C de manière à obtenir un dégagement régulier d'hydrogène.

En fin d'addition, on enlève le bain glacé et on laisse remonter lentement la température du milieu jusqu'à 32°C. On observe toujours un dégagement d'hydrogène. Lorsque la température est stabilisée vers 30°C, on chauffe lentement la masse réactionnelle jusqu'à 78°C, température que l'on maintient pendant 1 heure, jusqu'à cessation du dégagement gazeux.

On refroidit alors à 25°C puis on ajoute 22 g (0,10 mole) de bromure cuivrique anhydre (séché 1 heure à 150°C). La température s'élève spontanément 55°C puis on coule, en 30 minutes, une solution de 249 g (1,3 mole) de chloro-4 bromobenzène dans 300 ml de diméthylformamide anhydre (fraichement distillé).

Le mélange noir obtenu est chauffé vers 130 - 135°C et cette température est maintenue pendant 1 h 45.

Après refroidissement, on filtre la masse réactionnelle, on lave le précipité avec 200 ml d'éther éthylique, que l'on joint eu filtrat, et la phase organique ainsi obtenue est versée dans 500 ml d'eau distillée. Ce mélange est alors extrait par 3 x 200 ml d'éther. Cette nouvelle phase éthérée est de nouveeu levée avec 3 x 100 ml d'eau distillée, séchée par du sulfate de sodium anhydre et évaporée. Le résidu d'évaporation est soumis le distilletion sous pression réduite à l'aide d'une colonne de 20 cm remplie d'anneaux de Fenske et on recueille 244 g de chloro-4 (trifluoro-2,2,2 éthoxy) benzène ($Eb_{25}$ = 77 - 77,5°C) représentant un rendement de 89 %.

**Exemples 2 à 12**

Synthèse de (trifluoro-2,2,2 éthoxy)benzènes par condensation de trifluoroéthanolate de sodium sur les bromobenzènes correspondants en présence d'un sel de cuivre.

On utilise le même mode opératoire que celui décrit précédemment dans lequel le trifluoroéthanolate de sodium est préparé in situ par action de l'hydrure de sodium sur le trifluoroéthanol.

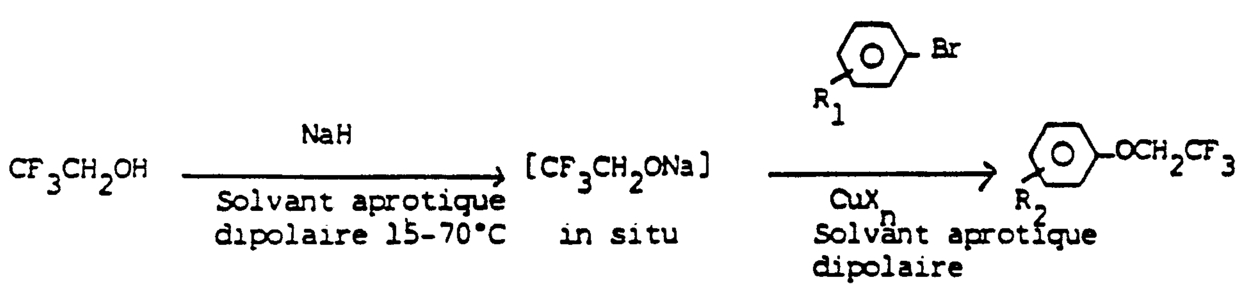


Les conditions et les résultats sont résumés dans le tableau I.

**Exemple 13**

Synthèse du chloro-4 (trifluoro-2,2,2 éthoxy)benzène à partir de dichloro-1,4 benzène et de trifluoroéthanol.

On emploie le même mode opératoire que dans l'exemple 1, en mettant en oeuvre:

| | |
|---|---|
| - p-dichlorobenzène | 73,5 g (0,5 mole) |
| - $CF_3CH_2OH$ | 53 g (0,53 mole) |
| - NaH à 50 % | 25,5 g (0,53 mole) |
| - $CuBr_2$ | 9 g (0,040 mole) |
| - DMF | 200 ml |

Au bout de 6 heures à 120 - 130°C on observe la formation de chloro-4 (trifluoro-2,2,2 éthoxy)benzène correspondant à un rendement d'environ 2 %.

**Exemple 14**

Synthèse du (trifluoro-2,2,2 éthoxy)benzène par action du bromobenzène, en présence de bromure cuivrique, sur du trifluoroéthylate de sodium isolé obtenu par action de la soude sur le trifluoroéthanol.

Dans un ballon à quatre tubulures muni d'un agitation mécanique centrale, d'une gaine thermométrique, d'une ampoule de coulée et d'une courte colonne Vigreux surmontée d'un condenseur avec décanteur de Dean-Stark pour recyclage de la phase inférieure, on charge 155 g (1,55 mole) de trifluoroéthanol et 100 ml de trichloro-1,1,2 trifluoro-1,2,2 éthane. En maintenant la température vers 15 - 20°C, on introduit lentement dans ce mélange une solution de 20 g (0,5 mole) de soude dans le minimum d'eau. Le milieu réactionnel est porté au reflux de l'azéotrope et l'eau distillée est décantée au fur et à mesure de sa formation. Lorsqu'on ne constate plus aucune décantation d'hétéroazéotrope, on joint le contenu du condenseur à celui du réacteur et on évapore l'ensemble sous pression réduite. Le trifluoroéthylate solide brut ainsi obtenu est ensuite mis en réaction sur le bromobenzène (78,5 g; 0,5 mole) au sein du diméthylformamide (500 ml) et en présence de 2 g (0,009 mole) de bromure cuivrique sec, de la manière indiquée dans l'exemple 1.

Au bout de 5 heures à 120°C, on récupère 26 de (trifluoro-2,2,2 éthoxy)benzène distillé.

**Exemple 15**

Synthèse du bis-(trifluoro-2,2,2 éthoxy)-1,4 benzène suivant la méthode décrite dans l'exemple 14 à partir de dibromo-1,4 benzène en utilisant du trifluoroéthylate de potassium obtenu par action de la potasse sur le trifluoroéthanol.

On opère a partir de 80 g (0,80 mole) de trifluoroéthanol que l'on traite par 23 g (0,41 mole) de potasse solide et on entraîne l'eau formée par azéotropie à l'aide de 70 ml de trichloro-1,1,2 trifluoro-1,2,2 éthane. Après cessation de libération d'eau, on rajoute au milieu 100 ml de diméthylformamide et on distille l'excès de trifluoroéthanol et le trichloro-1,1,2 trifluoro-1,2,2 éthane.

On poursuit la réaction comme indiqué dans l'exemple 10 en rajoutant 16 g (0,068 mole) de dibromo-1,4 benzène et 2 g (0,009 mole) de bromure cuivrique. Après chauffage 5 heures à 100°C, on obtient le bis-(trifluoro-2,2,2 éthoxy)-1,4 benzène avec un rendement de 34 %.

**Exemple 16**

Synthèse du bis-(trifluoro-2,2,2 éthoxy-1,4)benzène par action du dibromo-1,4 benzène, en présence de bromure cuivrique, sur du trifluoroéthylate de sodium obtenu par action du sodium sur le trifluoroéthanol.

On utilise le même appareillage que dans l'essai 1. On charge 100 g (1,00 mole) de trifluoroéthanol et on ajoute à 10 - 15°C, par petites portions, 4,7 g de sodium métallique bien décapé. Après la fin de l'addition du métal, on chauffe progressivement le milieu jusqu'à 50°C et on maintient cette température jusqu'à cessation du dégagement d'hydrogène. On ajoute ensuite 100 ml de diméthylformamide et on distille l'excès de trifluoroéthanol. La réaction est ensuite poursuivie comme indiqué dans l'exemple 15 en utilisant 16 g (0,068 mole) de dibromo-1,4 benzène et 2 g (0,009 mole) de bromure cuivrique.

Après 4 h 30 à 100°C, le rendement en bis-(trifluoro-2,2,2 éthoxy)-1,4 benzène isolé est de 75 %.

**Exemple 17**

Préparation du (trifluoro-2,2,2 éthoxy)-2 benzoate de méthyle à partir de trifluoroéthanol et de fluoro-2 benzoate de méthyle.

Dans un ballon à quatre tubulures équipé d'une agitation mécanique centrale, d'une ampoule de coulée, d'un réfrigérant ascendant et d'une gaine thermométrique, on charge 300 ml de diméthylformamide anhydre fraichement distillé et 26,5 g d'hydrure de sodium à 50 % en suspension dans l'huile de vaseline (soit 0,55 mole de NaH pur).

On refroidit vers 10° C cette suspension agitée l'aide d'un bain d'eau glacée, puis on coule, en 30 minutes, 55 g (0,55 mole) de trifluoroéthanol anhydre. Durant cette addition le milieu est maintenu vers 10 - 15° C et on observe un dégagement régulier d'hydrogène.

Après la fin de la coulée, on réchauffe le mélange réactionnel jusqu'à 50° C et on maintient cette température jusqu'à cessation du dégagement d'hydrogène.

On refroidit jusqu'à 35°C puis on coule, en 1 heure, 77 g (0,5 mole) de fluoro-2 benzoate de méthyle. La réaction est exothermique et il est nécessaire de refroidir pour maintenir le milieu vers 45° C. Cette température est maintenue pendant 2 heures après la fin de la coulée puis on refroidit à la température ambiante, on noie le mélange réactionnel dans l'eau et on extrait la phase aqueuse à l'éther.

La phase éthérée est ensuite évaporée et le résidu obtenu est distillé sous pression réduite. On obtient ainsi 81,5 g de (trifluoro-2,2,2 éthoxy)-2 benzoate de méthyle ($Eb_{20}$ = 132 - 135° C) représentant un rendement de 70 %.

**Exemples 18 à 22**

Synthèse de (trifluoro-2,2,2 éthoxy)benzènes par condensation de trifluoroéthylate de sodium sur des chloro et fluoro benzènes, activés envers la substitution nucléophile aromatique par des groupes électroattracteurs, au sein du diméthylformamide anhydre.

On utilise le même mode opératoire que celui décrit dans l'exemple précédent, le trifluoroéthylate de sodium étant préparé in situ par action de l'hydrure de sodium sur le trifluoroéthanol.

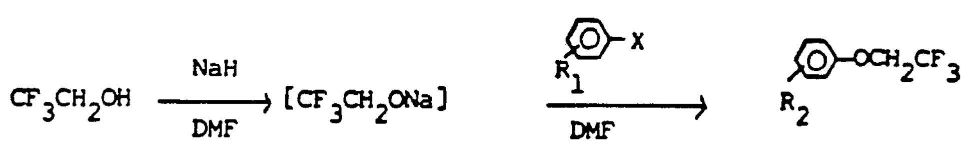


Les conditions et les résultats sont résumés dans le tableau II.

**Exemple 23**

Synthèse du (trifluoro-2,2,2 éthoxy)-4 benzonitrile par condensation du trifluoroéthanol sur le fluoro-4 benzonitrile au sein du trichlorobenzène-1,2,4 en présence de soude solide et d'un agent séquestrant.

Dans un ballon à quatre tubulures muni d'une agitation mécanique centrale, d'une gaine thermométrique, d'une ampoule de coulée et d'un réfrigérant ascendant, on charge 4,0 g (0,1 mole) de soude solide finement broyée, 250 ml de trichloro-1,2,4 benzène, 12,1 g (0,1 mole) de fluoro-4 benzonitrile et 1,62 g (0,005 mole) de tris-(dioxa-3,6 heptyl)amine, de formule $N(CH_2-CH_2-O-CH_2-CH_2-O-CH_3)_3$.

On coule alors, sous agitation et à température ambiante, 10 g (0,1 mole) de trifluoroéthanol et on porte le milieu agité à 145°C pendant 6 heures. La composition de la masse réactionnelle est suivie par chromatographie en phase gazeuse.

Après la fin de la réaction, on filtre le mélange réactionnel et on rince le précipité avec un peu de solvant que l'on joint au filtrat. Ce dernier est soumis à la distillation sous pression réduite à l'aide d'une colonne à bande tournante. On recueille ainsi 7,3 g de (trifluoro-2,2,2 éthoxy)-4 benzonicrile représentant un rendement de 36 %.

**Exemples 24 à 29**

Synthèse de (trifluoro-2,2,2 éthoxy)benzènes par condensation du trifluoroéthanol sur des chloro et fluorobenzènes activés envers la substitution nucléophile aromatique, au sein du trichloro-1,2,4 benzène et en présence de soude solide et de tris(dioxa-3,6 heptyl)amine (TDA-1).

On opère comme indiqué dans l'exemple 23.

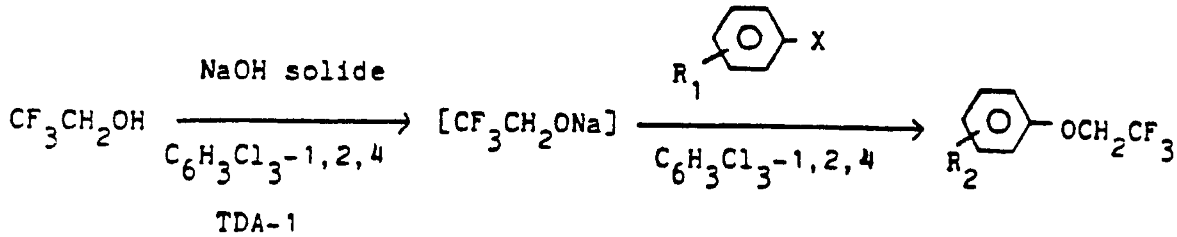


Les conditions et les résultats sont consignés dans le tableau III.

**Exemple 30**

Synthèse du (trifluoro-2,2,2 éthoxy)-4 benzoate de méthyle par condensation de trifluoroéthylate de sodium isolé sur le fluoro-4 benzoate de méthyle, au sein du trichloro-1,2,4 benzène et en présence de tris-(dioxa-3,6 heptyl)amine.

Dans un ballon à quatre tubulures muni d'une agitation mécanique centrale, d'une gaine thermométrique et d'un réfrigérant ascendant, on charge 50,8 g (0,508 mole) de trifluoroéthanol et on refroidit à 5°C. On ajoute alors, par petites portions, en 30 minutes, 3,76 g d'hydrure de sodium en dispersion à 50 dans l'huile de vaseline (soit 0,0784 mole de NaH pur) en maintenant la température entre 5 et 10°C. Après cette addition, on laisse la température remonter vers 20 - 25°C et on agite pendant 1 heure jusqu'à cessation du dégagement d'hydrogène. On évapore ensuite le trifluoroéthanol non réagi. Le résidu solide obtenu est ensuite rechargé dans le ballon avec 150 g de trichloro-1,2,4 benzène, 12,2 g (0,078 mole) de fluoro-4 benzoate de méthyle et 1,53 g (0,0047 mole) de tris-(dioxa-3,6 heptyl)amine. On porte ce mélange à 140°C pendant 5 heures sous agitation puis on le refroidit et on le filtre. Après distillation du filtrat, on récupère 8,83 g de (trifluoro-2,2,2 éthoxy)-4 benzoate de méthyle représentant un rendement de 48,3 %.

**Exemple 31**

Synthèse du chloro-4 (trifluoro-2,2,2 éthoxy)benzène à partir de chloro-4 phénol et de paratoluène sulfonate de trifluoro-2,2,2 éthyle au sein de la N-méthylpyrrolidone.

Dans un ballon de 250 ml à quatre tubulures, muni d'une agitation mécanique centrale, d'une gaine thermométrique, d'une ampoule de coulée et d'un réfrigérant ascendant, on charge 80 ml de N-méthylpyrrolidone anhydre fraichement distillée et 12,9 g (0,10 mole) de chloro-4 phénol. On agite et on charge, par petites portions, 5 g d'hydrure de sodium à 50 % en suspension dans l'huile de vaseline (soit 0,10 mole de NaH pur). On laisse la température monter spontanément vers 50°C tandis qu'on observe un dégagement régulier d'hydrogène. Lorsque ce dégagement diminue, on chauffe le milieu vers 130°C jusqu'à cessation de la formation d'hydrogène.

On refroidit ensuite vers 100°C et on coule alors, en 10 minutes, 28 g (0,11 mole) de paratoluène sulfonate de trifluoro-2,2,2 éthyle. On chauffe ensuite le mélange réactionnel 130 - 135°C et l'on maintient cette température pendant 4 heures 15 minutes.

Après refroidissement, on noie la masse réactionnelle dans l'eau distillée et on acidifie cette phase aqueuse jusqu'à pH = 1.

Cette phase aqueuse est ensuite extraite avec 3 x 100 ml d'éther éthylique; la phase éthérée est lavée avec 100 ml d'eau distillée, séchée et évaporée.

Le résidu est ensuite distillé sur colonne vigreux sous pression réduite.

On recueille ainsi 14 g de chloro-4 (trifluoro-2,2,2 éthoxy)benzène représentant un rendement de 67 %.

**Exemples 32 à 45**

Synthèse de (trifluoro-2,2,2 éthoxy)benzènes et de (trifluoro-2,2,2 éthylthio)benzènes à partir de phénols ou de thiophénols et d'esters sulfoniques ou carboxyliques du trifluoroéthanol, au sein d'un solvant aprotique dipolaire.

On opère comme décrit dans l'exemple 31.

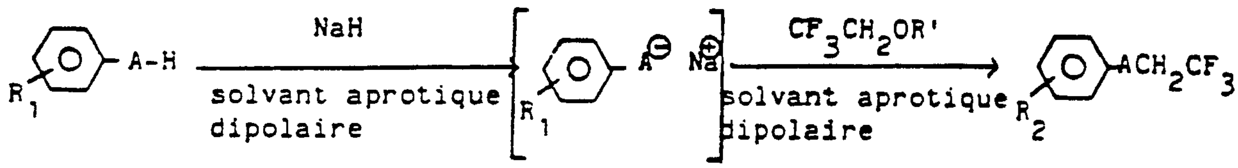


Les conditions et les résultats sont consignés dans les tableaux IV et IV (Suite).

**Exemple 46**

Synthèse du chloro-4 (trifluoro-2,2,2 éthoxy)benzène à partir de chloro-4 phénol et de trichlorométhanesulfonate de trifluoro-2,2,2 éthyle, au sein du trichloro-1,2,4 benzène et en présence de soude solide et de tris-(dioxa-3,6 heptyl)amine.

Dans un ballon de 500 ml équipé d'une agitation mécanique, d'un thermomètre et d'une petite colonne à distiller de type Vigreux, on charge:

- 200 ml de trichloro-1,2,4 benzène
- 4 g (0,1 mole) de soude finement broyée
- 1,6 g ($5.10^{-3}$ mole) de tris-(dioxa-3,6 heptyl)amine
- 12,9 g (0,1 mole) de parachlorophénol.

Cette suspension est agitée et portée à 100° C, température à partir de laquelle l'eau commence à distiller. Le milieu est maintenu entre 100 et 130° C jusqu'à cessation de la distillation de l'eau formée.

On refroidit alors vers 30°C et on introduit dans le mélange réactionnel 28,2 g (0,1 mole) de trichlorométhanesulfonate de trifluoro-2,2,2 éthyle.

On remplace la colonne à distiller par un réfrigérant ascendant et on porte, sous agitation, le milieu réactionel à 145° C, température que l'on maintient pendant 4 heures.

L'analyse chromatographique en phase vapeur, avec étalon interne, indique alors un rendement brut en chloro-4 (trifluoro-2,2,2 éthoxy)benzène de 58 %.

Le mélange obtenu après refroidissement est filtré et le filtrat est soumis à la distillation sous pression réduite à l'aide d'une colonne à bande tournante.

On recueille ainsi 11,6 g de chloro-4 (trifluoro-2,2,2 éthoxy)benzène représentant un rendement de 55 %. ($Eb_{25}$ = 77 - 77,5° C).

**Exemples 47 à 54**

Synthèse de (trifluoro-2,2,2 éthoxy)benzènes ou de (trifluoro-2,2,2 éthylthio)benzènes à partir de phénols ou de thiophénols et d'esters sulfoniques ou carboxyliques du trifluoroéthanol, au sein du trichloro-1,2,4 benzène et en présence de soude solide et de tris(dioxa-3,6 heptyl)amine.

Ces exemples sont résumés et détaillés dans le tableau V.

**Exemple 55**

Chloration du chloro-4 (trifluoro-2,2,2 éthoxy)benzène au sein du tétrachlorure de carbone et en présence de radiations.

Dans un réacteur cylindrique de deux litres utiles, muni d'une agitation, d'un réfrigérant ascendant, d'un dispositif de mesure de température, d'un tube plongeant poreux pour introduction de gaz et d'une gaine centrale dans laquelle est placée une lampe à décharge émettant autour de 400 nm, on charge deux litres d'une solution contenant 379 g (1,8 mole) de chloro-4(trifluoro-2,2,2 éthoxy)benzène pur dans du tétrachlorure de carbone.

On porte la solution irradiée au reflux sous courant d'azote. Une fois le reflux atteint, on arrête l'introduction d'azote et on la remplace par une introduction de chlore gazeux (débit initial 520 g/h) de manière à saturer de chlore la solution bouillante irradiée.

La chloration photochimique dure 3 h 30 minutes. Son évolution est suivie par chromatographie en phase gazeuse.

Lorsqu'elle est terminée, on maintient le milieu réactionnel au reflux sous irradiation et on remplace le chlore par de l'azote jusqu'à dégazage complet du liquide. On refroidit et on arrête l'irradiation.

On distille le tétrachlorure de carbone sous pression atmosphérique puis le chloro-4 (dichloro-1,1 trifluoro-2,2,2 éthoxy)benzène sous pression réduite ($Eb_{18}$ = 103° C).

On recueille ainsi 463 g de produit représentant un rendement de 92 %.

**Exemple 56**

Chloration du chloro-4(trifluoro-2,2,2 éthoxy)benzène en l'absence de solvant et en présence de radiations.

La même réaction est effectuée suivant le processus ci-dessus, mais à 150° C en l'absence de tétrachlorure de carbone. Elle conduit, en 2 h 30 minutes, à un rendement en chloro-4 (dichloro-1,1 trifluoro-2,2,2 éthoxy)benzène distillé de 84 %.

**Exemples 57 à 70**

Chloration de (trifluoro-2,2,2· éthoxy)benzènes et de (trifluoro-2,2,2 éthylthio)benzènes en présence de radiations.

Ces exemples sont résumés et détaillés dans le tableau VI et le tableau VI suite.

**Exemple 71**

Chloration du chloro-4 (trifluoro-2,2,2 éthoxy)benzène en l'absence de solvant et en présence de trichlorure de phosphore comme catalyseur.

Dans un réacteur de 100 ml muni d'une agitation par barreau aimanté, d'un réfrigérant ascendant, d'un dispositif de mesure de température et d'un tube plongeant poreux pour introduction de gaz, on charge 50 g de chloro-4(trifluoro-2,2,2 éthoxy)benzène et 1 g de trichlorure de phosphore ($PCl_3/ArOCH_2CF_3$ = 0,036 mole/mole). On chauffe le milieu réactionnel sous azote jusqu'à 150° C puis on remplace l'introduction d'azote par celle du chlore. La température est maintenue entre 150 et 160° C durant toute la chloration, dont l'avancement est suivi par chromatographie en phase gazeuse. Cette chloration dure 8 h 10 minutes. Lorsqu'elle est terminée, on arrête l'introduction de chlore et, toujours vers 150° C, on dégaze la masse réactionnelle avec de l'azote pendant 30 minutes. Puis le mélange est refroidi et le produit désiré est distillé. Le rendement en chloro-4(dichloro-1,1 trifluoro-2,2,2 éthoxy)benzène s'établit à 78 %.

**Exemple 72**

Chloration du chloro-4(trifluoro-2,2,2 éthoxy)benzène en l'absence de solvant et en présence de radiations et de trichlorure de phosphore.

On opère comme dans l'exemple 56 mais on ajoute préalablement à la chloration 3,6 % mole de trichlorure de phosphore par rapport au substrat organique.

La chloration dure 4 h 20 minutes à 150 - 160° C et fournit le chloro-4(dichloro-1,1 trifluoro-2,2,2 éthoxy)benzène avec un rendement de 88 %.

**Exemple 73**

p-chloro(pentafluoroéthoxy)benzène

Dans un réacteur inox de 250 ml agité par barreau aimanté, on introduit successivement, sous atmosphère inerte ($N_2$):

- 3 g (0,01 mole) de $SbCl_5$ anhydre
- 28 g (0,1 mole) de p-chloro (1,1-dichloro 2,2,2-trifluoroéthoxy)benzène
- 25 g (1,25 mole) de HF anhydre.

Le mélange réactionnel est porté, sous agitation, à 120°C pendant 3 h 15 mn. La pression s'élève jusqu'à 26 bars. Après réaction, le réacteur est refroidi vers 0°C, décomprimé (on laisse échapper l'acide chlorhydrique). Le mélange réactionnel obtenu est coulé sur 85 g de glace pilée. Après extraction par trois fois 100 ml de $CH_2Cl_2$, lavage des phases organiques à l'eau permutée (100 ml) et filtration, on mesure par titrage infra-rouge environ 24 g de p-chloro(pentafluoroéthoxy)benzène, soit un rendement analytique d'environ 97,5 %. Par chromatographie en phase gazeuse, on note l'absence du composé de départ.

Après évaporation du solvant, on récupère 17,2 g de parachloro(pentafluoroéthoxy)benzène (rendement : 70 %).

**Essai comparatif**

On utilise le même mode opératoire qu'à l'exemple 73 mais en supprimant $SbCl_5$.

Après 3 heures de réaction, on récupère intégralement le produit de départ et on n'obtient aucune trace de p-chloro(pentafluoroéthoxy)benzène.

**Exemple 74**

p-nitro(pentafluoroéthylthio)benzène

On opère de manière identique à l'exemple 73 avec les conditions et composés suivants:

| | |
|---|---|
| - p-nitro(1,1-dichloro-2,2,2-trifluoroéthylthio)benzène: | 5 g - 0,017 mole |
| - HF anhydre: | 10 g - 0,5 mole |
| - $SbCl_5$: | 0,8 g - 0,0027 mole |
| - Température: | 100°C |
| - Durée: | 5 heures |

Les analyses par chromatographie phase gazeuse (normalisation à 100) et spectrométrie de masse montrent la présence de 50 % de p-nitro(pentafluoroéthylthio)benzène.

**Exemple 75**

(Pentafluoroéthoxy)-benzène

On opère de manière identique à l'exemple 73 avec les conditions et composés suivants:

| | |
|---|---|
| - (1,1-dichloro-2,2,2-trifluoroéthoxy)benzène: | 10 g - 0,04 mole |
| - HF anhydre: | 20 g - 1 mole |
| - $SbCl_5$: | 0,6 g - 0,002 mole |
| - Température: | 130°C |
| - Durée: | 4 heures |

Les analyses par chromatographie phase gazeuse (normalisation à 100) et spectrométrie de masse montrent la présence de 76 % de (pentafluoroéthoxy)benzène.

**Exemple 76**

m-chloro(pentafluoroéthylthio)benzène

On opère de manière identique à l'exemple 73 avec les conditions et composés suivants:

| | |
|---|---|
| - m-chloro(1,1-dichloro-2,2,2-trifluoroéthylthio)benzène: | 3 g - 0,01 mole |
| - HF anhydre: | 10 g - 0,5 mole |
| - $SbCl_5$: | 0,3 g - 0,001 mole |
| - Température: | 150°C |
| - Durée: | 6 heures |

Les analyses par chromatographie phase gazeuse (normalisation à 100) et spectrométrie de masse montrent la présence de 22 % de m-chloro (pentafluoroéthylthio)benzène.

**Exemple 77**

p-chloro(pentafluoroéthoxy)benzène

On opère de manière identique à l'exemple 73 avec les conditions et composés suivants:

| | |
|---|---|
| - p-chloro 1,1 dichloro-2,2,2-trifluoroéthoxybenzène: | 42 g - 0,15 mole |
| - HF anhydre: | 50 g - 2,5 mole |
| - $BF_3$: | 10 bars à 20° C |
| - Température: | 100° C |
| - Durée: | 3 h 45 mn |

Les analyses par chromatographie phase gazeuse (normalisation à 100) et spectrométrie de masse montrent la présence de 39,5 % de p-chloro (pentafluoroéthoxy)benzène.

**Exemple 78**

p-chloro(pentafluoroéthoxy)benzène

On opère de manière identique à l'exemple 73 avec les conditions et composés suivants:

| | |
|---|---|
| - p-chloro(1,1-dichloro-2,2,2-trifluoroéthoxy)benzène: | 42 g - 0,15 mole |
| - HF anhydre: | 50 g - 2,5 mole |
| - $SnCl_4$: | 5,8 g - 0,022 mole |
| - Température: | 150° C |
| - Durée: | 6 heures |

Les analyses par chromatographie phase gazeuse (normalisation à 100) et spectrométrie de masse montrent la présence de 38 % de p-chloro(pentafluoroéthoxy)benzène.

**Exemple 79**

p-chloro(pentafluoroéthoxy)benzène

On opère de manière identique à l'exemple 73 avec les conditions et composés suivants:

| | |
|---|---|
| - p-chloro(1,1-dichloro-2,2,2-trifluoroéthoxy)benzène: | 10,5 g - 0,04 mole |
| - HF anhydre: | 40 g - 2 moles |
| - $TiCl_4$: | 1 g - 0,005 mole |
| - Température: | 150° C |
| - Durée: | 22 heures |

Les analyses par chromatographie phase gazeuse (normalisation à 100) et spectrométrie de masse montrent la présence de 18 % de p-chloro(pentafluoroéthoxy)benzène.

**Exemple 80**

Synthèse de bis-(trifluoro-2,2,2 éthoxy) 1,4 benzène par action du dibromo-1,4 benzène en présence de bromure cuivrique, sur du trifluoroéthylate de sodium obtenu par action de l'amidure de sodium sur le trifluoroéthanol.

On utilise le même appareillage que dans l'exemple 1, on charge 100 ml de toluène puis 0,5 mole d'amidure de sodium. On coule lentement une mole de trifluoroéthanol.

La température augmente progressivement jusqu'à 30° C. Après la fin du dégagement d'ammoniac, on évapore le toluène et le trifluoroéthanol en excès. On rajoute 50 ml de DMF puis 2 g de bromure cuivrique. On coule 16 g (0,067 mole) de dibromo-1,4 benzène dissous dans 50 ml de DMF. La réaction est poursuivie pendant 3 heures à 100° C. Après hydrolyse du mélange réactionnel par HCl aqueux, puis filtration, on récupère 13 g de bis(trifluoro-2,2,2 éthoxy) 1,4-benzène représentant un rendement de 70 %.

**Tableau I**

| N° | $R_1$ | Solvant | $\frac{CH_3CH_2OH}{NaH}$ moles/moles | $\frac{NaH}{Ar\text{-}Br}$ moles/moles | $CuX_n$ | $\frac{CuX_n}{Ar\text{-}Br}$ moles/moles |
|---|---|---|---|---|---|---|
| 2 | Cl-4 | DMF | 1 | 1,04 | $CuBr_2$ | 0,038 |
| 3 | Cl-4 | DMF | 1,05 | 1 | $CuBr_2$ | 0,025 |
| 4 | H | DMF | 1 | 1,05 | $CuBr_2$ | 0,078 |
| 5 | H | DMF | 1,05 | 0,90 | $CuBr_2$ | 0,030 |
| 6 | H | DMF | 1 | 0,5 | $CuBr_2$ | 0,011 |
| 7 | H | DMF | 1,8 | 0,53 | $CuBr_2$ | 0,020 |
| 8 | H | DMF | 1 | 0,91 | $CuBr_2$ | 0,020 |
| 9 | Br-4 | DMF | 1 | 2,2 | $CuBr_2$ | 0,179 |
| 10 | Br-4 | DMF | 1,4 | 3 | $CuBr_2$ | 0,074 |
| 11 | Br-4 | DMF | 1,4 | 3 | $CuBr_2$ | 0,032 |
| 12 | Br-4 | DMF | 1,4 | 3 | CuCl | 0,074 |

**Tableau I** (suite)

| N° | T°C | Durée h | $R_2$ | Rdt |
|---|---|---|---|---|
| 2 | 130 - 140°C | 3 h | Cl-4 | 64 % |
| 3 | 120°C | 31 h | Cl-4 | 74 % |
| 4 | 120 - 125°C | 3 h 40 mn | H | 87 % |
| 5 | 120°C | 9 h | H | 68 % |
| 6 | 120°C | 5 h | H | 55,5 % |
| 7 | 120°C | 5 h | H | 56 % |
| 8 | 120°C | 5 h | H | 38 % |
| 9 | 130 - 135°C | 4 h 15 mn | Br-4 | 28 % |
| | | | $CF_3CH_2O$-4 | 41 % |
| 10 | 100°C | 0 h 30 mn | $CF_3CH_2O$-4 | 97 % |
| 11 | 100°C | 2 h | $CF_3CH_2O$-4 | 98 % |
| 12 | 100°C | 2 h | $CF_3CH_2O$-4 | 92 % |

DMF = N,N-diméthylformamide
NMP = N-méthylpyrrolidone

**Tableau II**

| N° | $R_1$ | X | $\frac{CF_3CH_2OH}{NaH}$ (moles/moles) | $\frac{NaH}{ArX}$ (moles/moles) | T°C | Durée h | $R_2$ | Rdt |
|---|---|---|---|---|---|---|---|---|
| 18 | $O_2N$-4 | F | 1 | 1,06 | 45°C | 1 h | $O_2N$-4 | 99 % |
| 19 | $O_2N$-4 | F | 1 | 1,06 | 45°C | 1 h | $O_2N$-4 | 92 % |
| 20 | $F_3C$-4 | F | 1 | 1,05 | 75°C | 2 h | $F_3C$-4 | 81 % |
| 21 | $F_3C$-4 | Cl | 1 | 1,1 | 140°C | 8 h 30 mn | $F_3C$-4 | 1 % |
| 22 | p-F-$C_6H_4$-CO-4 | F | 1 | 2,2 | 50°C | 2 h | p-$CF_3CH_2O$-$C_6H_4$-CO-4 | 55 % |

**Tableau III**

| N° | $R_1$ | X | $\frac{CF_3CH_2OH}{NaOH}$ (moles/moles) | $\frac{NaOH}{ArX}$ (moles/moles) | $\frac{TDA\text{-}1}{ArX}$ (moles/moles) | T°C | Durée h | $R_2$ | Rdt % |
|---|---|---|---|---|---|---|---|---|---|
| 24 | $O_2N$-4 | F | 1 | 1 | 0,05 | 147°C | 4 h | $O_2N$-4 | 82 |
| 25 | $O_2N$-4 | F | 1 | 1 | 0,05 | 100°C | 6 h | $O_2N$-4 | 70 |
| 26 | $O_2N$-4 | Cl | 1 | 1 | 0,05 | 130°C | 4 h | $O_2N$-4 | 42 |
| 27 | $O_2N$-4 | Cl | 1 | 1 | 0,05 | 140°C | 7 h | $O_2N$-4 | 34 |
| 28 | $F_3C$-4 | F | 1 | 1 | 0,10 | 145°C | 26 h | $F_3C$-4 | 44,5 |
| 29 | p-F-$C_6H_4$-CO-4 | F | 1 | 2 | 0,12 | 135 - 170°C | 10 h | p-F-$C_6H_4$-CO-4 | 47 |
| | | | | | | | | p-$CF_3CH_2O$-$C_6H_4$-CO-4 | 25 |

TDA-1 = tris-(dioxa-3,6 heptyl)amine

**Tableau IV**

| N° | $R_1$ | A | $\frac{AR\text{-}AH}{NaH}$ moles/moles | $\frac{CF_3CH_2OR'}{Ar\text{-}AH}$ moles/moles | Solvant | T°C | Durée |
|---|---|---|---|---|---|---|---|
| 32 | Cl-4 | O | 1 | 1,2 | HMPT | 140°C | 6 h |
| 33 | Cl-4 | O | 1 | 1,2 | HMPT | 140°C | 24 h |
| 34 | Cl-4 | O | 1 | 1,2 | NMP | 140°C | 24 h |
| 35 | Cl-4 | O | 1 | 1,05 | HMPT | 120°C<br>+140°C | 2 h<br>5 h |
| 36 | Cl-4 | O | 1 | 1,08 | HMPT | 120°C | 2 h 30 mn |
| 37 | Cl-4 | O | 1 | 1,05 | HMPT | 130°C | 0 h 15 mn |
| 38 | Cl-4 | O | 1 | 1,1 | NMP | 130 - 140°C | 6 h |
| 39 | H | O | 1 | 1,05 | NMP+ sulfolane (150/100) | 130 - 140°C | 3 h |
| 40 | $CH_3O$-4 | O | 1 | 1,1 | NMP | 135°C | 3 h |
| 41 | H | S | 1 | 1,1 | NMP | 130 - 140°C | 4 h |
| 42 | Cl-3 | S | 1 | 1,06 | NMP | 80°C | 4 h |
| 43 | $O_2N$-4 | S | 1 | 1 | NMP | 80°C | 4 h |
| 44 | 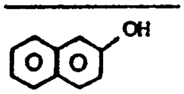 | | 1 | 1,1 | NMP | 140 - 150°C | 3 h |
| 45 | 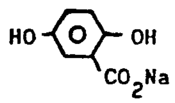 | | 0,5 | 3 | HMPT | 140°C | 6 h 30 mn |

**Tableau IV** (suite)

| N° | R' | $R_2$ | Rdt |
|---|---|---|---|
| 32 | $CH_3SO_2$ | Cl-4 | 28 % |
| 33 | $CH_3SO_2$ | Cl-4 | 38 % |
| 34 | $CH_3SO_2$ | Cl-4 | 21 % |
| 35 | $CF_3CO$ | Cl-4 | 58 % |
| 36 | $CCl_3SO_2$ | Cl-4 | 66 % |
| 37 | TsO | Cl-4 | 75 % |
| 38 | $ClSO_2$ | Cl-4 | 25 % |
| 39 | TsO | H | 72 % |
| 40 | TsO | $CH_3O$-4 | 75 % |
| 41 | $CH_3SO_2$ | H | 66 % |
| 42 | $CH_3SO_2$ | Cl-3 | 72 % |
| 43 | TsO | $O_2N$-4 | 58 % |
| 44 | TsO | 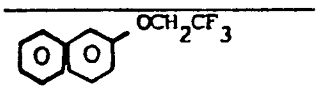 | 38 % |
| 45 | $CCl_3SO_2$ | 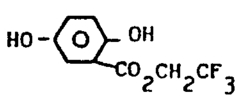 | 35 % |
| | | 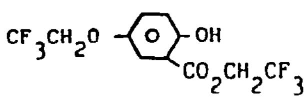 | 4 % |
| | | 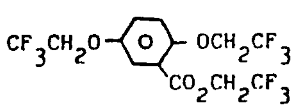 | 7 % |

NMP = N-méthylpyrrolidone HMPT = Hexaméthylphosphorotriamide TsO = p-toluènesulfonyle

**Tableau V**

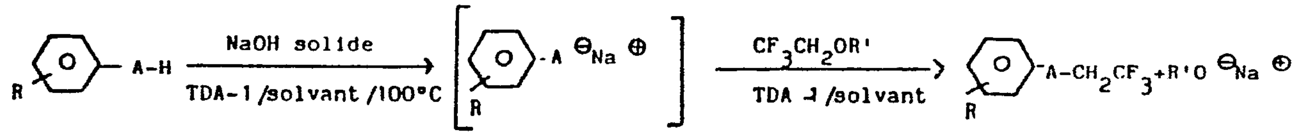


| R | A | Solvant | R' | $\frac{Ar\text{-}A\text{-}H}{CH_3CH_2OR'}$ mole/mole | $\frac{NaOH}{Ar\text{-}AH}$ mole/mole | $\frac{TDA\text{-}1}{Ar\text{-}A\text{-}H}$ % moles | [Ar-A-H] moles/l |
|---|---|---|---|---|---|---|---|
| H | O | TCB | TsO | 1 | 1 | 5 | 0,4 |
| $CH_3$-4 | O | TCB | $CCl_3SO_2$ | 0,7 | 1 | 4 | 0,5 |
| $CH_3O$-4 | O | TCB | $CH_3SO_2$ | 1 | 1 | 5 | 0,33 |
| Cl-2 | O | TCB | $CCl_3SO_2$ | 1 | 1 | 5 | 0,5 |
| Cl-4 | O | TCB | $CCl_3SO_2$ | 1,2 | 0,85 | 5 | 0,5 |
| Cl-4 | O | TCB | TsO | 1 | 1 | 5 | 0,5 |
| H | S | TCB | $CF_3CO$ | 1 | 1 | 7,6 | 0,7 |
| $C_{10}H_7OH$-2 | | TCB | TsO | 1 | 1 | 15 | 0,5 |

**Tableau V** (suite)

| °C | Durée (h) | Rdt Ar-A-$CH_2CF_3$ % |
|---|---|---|
| 170°C | 6 | 31 % brut<br>21 % distillé |
| 145°C | 4 | 59 % distillé |
| 140°C | 6 | 21 % brut |
| 145°C | 4 | 61 % distillé |
| 145°C | 4 | 58 % brut<br>55 % distillé |
| 150°C<br>+186°C | 6<br>6 | 27 % brut<br>57 % brut |
| +186°C | 2 | 70 % brut |
| 120 - 140°C | 12 | 34 % distillé |
| 140°C<br>+160°C | 12<br>+6 | 20 % cristallisé |

TCB = trichloro-1,2,4 benzène TsO = $CH_3$ 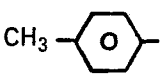 $SO_2$ TDA-1 = $N(CH_2\text{-}CH_2\text{-}O\text{-}CH_2\text{-}CH_2\text{-}O\text{-}CH_3)_3$

**Tableau VI**

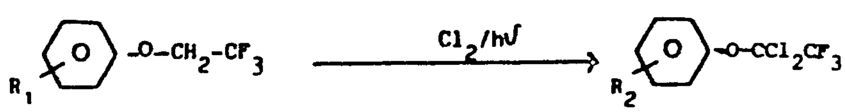


| Essai | $R_1$ | Solvant | [Ar-O-$CH_2CF_3$] | T°C | Durée | $R_2$ | Rdt distillé |
|---|---|---|---|---|---|---|---|
| 57 | Cl-2 | Sans | - | 150 | 4 h | Cl-2 | 75 % |
| 58 | H | $CCl_4$ | 2 moles/l | 78 - 81 | 24 h | H | 61 % |
| 59 | $CH_3$-4 | TCB | 1,14 moles/l | 115 | 9 h | $CCl_3$-4 | 45 % |
| 60 | $CH_3O$-4 | $CCl_4$ | 1 mole/l | 78 - 80 | 8 h | $CCl_3O$-4 | 44 % |
| 61 | $CF_3CH_2$-O-4 | $CCl_4$ | 0,5 mole/l | 78 - 80 | 6 h | $CF_3$-$CCl_2$-O-4 | 58 % |
| 62 | $CF_4$-4 | sans | - | 120 - 130 | 5 h | $CF_3$-4 | 73 % |
| 63 | NC-4 | $CCl_4$ | 1 mole/l | 80 | 5 h | NC-4 | 64 % |
| 64 | $CH_3O_2C$-4 | ODCB | 3 moles/l | 160 - 170 | 12 h | ClOC-4 | 85 % |
| 65 | $CH_3O_2C$-2 | ODCB | 3 moles/l | 160 - 170 | 16 h | ClOC-2 | 65 % |
| 66 | p-$CF_3CH_2OC_6H_4CO$-4 | $CCl_4$ | 0,5 mole/l | 78 - 80 | 20 h | p-$CF_3$-$CCl_2$-$OC_6$-$H_4CO$-4 | 54 % |
| 67 | $C_{10}H_7OCH_2CF_3$-2 | $CCl_4$ | 1 mole/l | 80 | 20 h | $C_{10}H_7OCCl_2CF_3$-2 | 57 % |

TCB = Trichlorobenzène-1,2,4 ODCB = Orthodichlorobenzène $C_{10}H_7$ = naphtyl

**Tableau VI** (suite)

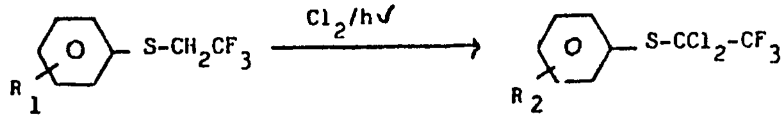


| Essai | $R_1$ | Solvant | $[Ar\text{-}S\text{-}CH_2CF_3]$ | T°C | Durée | $R_2$ | Rdt distillé |
|---|---|---|---|---|---|---|---|
| 68 | H | $CCl_4$ | 1 mole/l | 78 - 80 | 20 h | H | 61 % |
| 69 | Cl-3 | $CCl_4$ | 1 mole/l | 78 - 80 | 19 h | Cl-3 | 56 % |
| 70 | $O_2N$-4 | $CCl_4$ | 1 mole/l | 78 - 80 | 23 h 30 | $O_2N$-4 | 90 % |

TCB = Trichlorobenzène-1,2,4 ODCB= Orthodichlorobenzène

**Revendications**

1. Procédé de préparation de dérivés (pentafluoroéthoxy) et (pentafluoroéthylthio) benzéniques caractérisé en ce que:

- dans une première étape on fait réagir, soit un halogénobenzène avec du trifluoroéthanol dans un solvant aprotique en présence d'une base forte et en présence éventuellement d'un sel de cuivre et/ou d'un agent complexant, soit un dérivé phénolique ou thiophénolique avec un dérivé de formule générale (I)

$$CF_3\text{-}CH_2\text{-}O\text{-}R' \qquad (I)$$

dans laquelle R' représente un substituant choisi parmi le groupe comprenant le trifluoracétyle, le méthane sulfonyle, le paratoluène sulfonyle, le trichlorométhane sulfonyle, le chlorosulfonyle, le trifluorométhanesulfonyle, en présence d'une base alcaline forte, d'un solvant aprotique, et éventuellement d'un agent complexant;

- dans une deuxième étape, on procède à la chloration du dérivé issu de la première étape, à l'aide de chlore gazeux en présence de radiations et/ou de trichlorure de phosphore;

- dans une troisième étape, on procède à la fluoration du produit issu de la deuxième étape dans l'acide fluorhydrique liquide anhydre en présence d'un acide de Lewis.

2. Procédé selon la revendication 1, caractérisé en ce que au cours de la première étape, on fait réagir un bromo ou un iodobenzène avec du trifluoroéthanolate en présence de bromure ou de chlorure de cuivre en solution dans un solvant aprotique dipolaire.

3. Procédé selon la revendication 1, caractérisé en ce que au cours de la première étape, on fait réagir un halogénobenzène activé par un substituant électroattracteur avec du trifluoroéthanol en présence d'une base dans un solvant aprotique et éventuellement en présence d'un agent complexant.

4. Procédé selon les revendications 2 ou 3, caractérisé en ce que le rapport molaire du trifluoroéthanol au composé halogéné est compris entre 0,5 et 5.

5. Procédé selon la revendication 1, caractérisé en ce que le composé de formule (I) est choisi parmi le paratoluène sulfonate et le trichlorométhane sulfonate de trifluoro-2,2,2 éthyle.

6. Procédé selon la revendication 1, caractérisé en ce que le rapport molaire du phénol ou thiophénol au composé de formule (I) est compris entre 0,5 et 5, et de préférence entre 0,7 et 1,5.

7. Procédé selon la revendication 1, caractérisé en ce que la base est choisie parmi l'hydrure de sodium, la potasse, la soude et le sodium.

8. Procédé selon la revendication 1, caractérisé en ce que l'agent complexant répond à la formule générale (II)

$$N\text{-}[CHR_1\text{-}CHR_2\text{-}O\text{-}(CHR_3\text{-}CHR_4\text{-}O)_n\text{-}R_5]_3 \qquad (II)$$

dans laquelle n est un nombre entier supérieur ou égal à 0 et inférieur ou égal à 10 ($0 \leqslant n \leqslant 10$), $R_1$, $R_2$, $R_3$ et $R_4$ identiques ou différents, représentent un atome d'hydrogène ou un radical alkyle ayant de 1 à 4 atomes de carbone et $R_5$ représente un radical alkyle ou cycloalkyle ayant de 1 à 12 atomes de carbone, un radical phényle ou un radical de formule:

$$-C_mH_{2m}C_6H_5 \text{ ou } C_mH_{2m+1}C_6H_4\text{-},$$

m étant compris entre 1 et 12.

9. Procédé selon les revendications 1 à 8, caractérisé en ce que le rapport molaire du dérivé de formule (II) à l'halogénobenzène, au phénol ou au thiophénol est compris entre 0,01 et 0,2.

10. Procédé selon la revendication 1, caractérisé en ce que au cours de la deuxième étape, on fait réagir les composés issus de la première étape avec du chlore gazeux en présence de rayonnements.

11. Procédé selon la revendication 10, caractérisé en ce que au cours de la deuxième étape, on rajoute un solvant choisi parmi le tétrachlorure de carbone et les chlorobenzènes et de préférence le tétrachlorure de carbone.

12. Procédé selon la revendication 10, caractérisé en ce que la température de réaction est comprise entre 70 et 200° C.

13. Procédé selon les revendications 11 et 12, caractérisé en ce que la température de réaction est comprise éntre 70 et 85° C.

14. Procédé selon la revendication 1, caractérisé en ce que l'acide de Lewis est choisi parmi le pentachlorure d'antimoine et le trifluorure de bore.

15. Procédé selon les revendications 1 ou 14, caractérisé en ce que le rapport molaire de l'acide de Lewis au composé issu de l'étape 2 est compris entre 1 et 20 % et de préférence entre 5 et 15 %.

16. Procédé selon la revendication 1, caractérisé en ce que le rapport molaire du composé issu de l'étape 2 à l'acide fluorhydrique est compris entre 5 et 50 et de préférence entre 10 et 30.

**Patentansprüche**

1. Verfahren zur Herstellung von (Pentafluorethoxy)- und (Pentafluorethylthio)-Benzolderivaten, dadurch gekennzeichnet, daß:

- man in einem ersten Schritt entweder ein Halogenbenzol mit Trifluorethanol in einem aprotischen Lösungsmittel in Gegenwart einer starken Base und gegebenenfalls in Anwesenheit eines Kupfersalzes und/oder eines Komplexierungsmittels, oder ein Phenolderivat oder Thiophenolderivat mit einer Verbindung der allgemeinen Formel (I) reagieren läßt

$$CF_3\text{-}CH_2\text{-}O\text{-}R' \qquad (I)$$

in der R' einen Substituenten ausgewählt aus der Gruppe umfassend Trifluoracetyl, Methansulfonyl, Paratoluolsulfonyl, Trichlormethansulfonyl, Chlorsulfonyl, Trifluormethansulfonyl, darstellt, in Gegenwart einer starken Alkalibase, eines aprotischen Lösungsmittels und gegebenenfalls eines Komplexierungsmittels;

- man in einem zweiten Schritt die Chlorierung der im ersten Schritt erhaltenen Verbindung mit Hilfe von gasförmigem Chlor bei gleichzeitiger Bestrahlung und/oder Anwesenheit von Phosphortrichlorid durchführt;

- man in einem dritten Schritt die Fluorierung des im zweiten Schritt erhaltenen Produkts in flüssiger wasserfreier Fluorwasserstoffsäure in Gegenwart einer Lewis-Säure vornimmt.

2. Verfahren nach Anspruch 1, dadurch gekennzeichnet, daß man im Verlauf des ersten Schritts ein Brom- oder ein Jodbenzol mit Trifluorethanolat in Gegenwart von Kupferbromid oder -chlorid in Lösung in einem dipolaren aprotischen Lösungsmittel reagieren läßt.

3. Verfahren nach Anspruch 1, dadurch gekennzeichnet, daß man im Verlauf des ersten Schritts ein durch einen elektronenanziehenden Substituenten aktiviertes Halogenbenzol mit Trifluorethanol in Gegenwart einer Base in einem aprotischen Lösungsmittel und gegebenenfalls in Gegenwart eines Komplexierungsmittels reagieren läßt.

4. Verfahren nach den Ansprüchen 2 oder 3, dadurch gekennzeichnet, daß das molare Verhältnis von Trifluorethanol zur Halogenverbindung zwischen 0,5 und 5 beträgt.

5. Verfahren nach Anspruch 1, dadurch gekennzeichnet, daß die Verbindung der Formel (I) ausgewählt wird aus Paratoluolsulfonat und 2,2,2-Trifluor-ethyltrichlormethansulfonat.

6. Verfahren nach Anspruch 1, dadurch gekennzeichnet, daß das molare Verhältnis des Phenols oder Thiophenols zur Verbindung der Formel (I) zwischen 0,5 und 5 und vorzugsweise zwischen 0,7 und 1,5 beträgt.

7. Verfahren nach Anspruch 1, dadurch gekennzeichnet, daß die Base ausgewählt wird aus Natriumhydrid, Pottasche, Soda und Natrium.

8. Verfahren nach Anspruch 1, dadurch gekennzeichnet, daß das Komplexierungsmittel der allgemeinen Formel (II) entspricht

$$N\text{-}[CHR_1\text{-}CHR_2\text{-}O\text{-}(CHR_3\text{-}CHR_4O)_n\text{-}R_5]_3 \qquad (II)$$

in der n eine ganze Zahl größer oder gleich 0 und kleiner oder gleich 10 ist ($0 \leqslant n \leqslant 10$), $R_1$, $R_2$, $R_3$ und $R_4$ identisch oder verschieden sind und jeweils ein Wasserstoffatom oder einen Alkylrest mit 1 bis 4 Kohlenstoffatomen darstellen und $R_5$ einen Alkylrest oder Cycloalkylrest mit 1 bis 12 Kohlenstoffatomen, einen Phenylrest oder einen Rest der Formel:

$C_mH_{2m}C_6H_5$ oder $C_mH_{2m+1}$ $C_6H_4$-

darstellt, wobei m zwischen 1 und 12 liegt.

9. Verfahren nach den Ansprüchen 1 bis 8, dadurch gekennzeichnet, daß das molare Verhältnis der Verbindung der Formel (II) zum Halogenbenzol, zum Phenol oder zum Thiophenol zwischen 0,01 und 0,2 beträgt.

10. Verfahren nach Anspruch 1, dadurch gekennzeichnet, daß man im Verlauf des zweiten Schritts die im ersten Schritt erhaltenen Verbindungen mit gasförmigem Chlor bei gleichzeitiger Bestrahlung reagieren läßt.

11. Verfahren nach Anspruch 10, dadurch gekennzeichnet, daß man im Verlauf des zweiten Schritts ein Lösungsmittel zugibt ausgewählt aus Tetrachlorkohlenstoff und den Chlorbenzolen und vorzugsweise Tetrachlorkohlenstoff.

12. Verfahren nach Anspruch 10, dadurch gekennzeichnet, daß die Temperatur der Reaktion zwischen 70 und 200°C liegt.

13. Verfahren nach den Ansprüchen 11 und 12, dadurch gekennzeichnet, daß die Temperatur der Reaktion zwischen 70 und 85°C liegt.

14. Verfahren nach Anspruch 1, dadurch gekennzeichnet, daß die Lewis-Säure ausgewählt wird aus Antimonpentachlorid und Bortrifluorid.

15. Verfahren nach den Ansprüchen 1 oder 14, dadurch gekennzeichnet, daß das molare Verhältnis der Lewis-Säure zur Verbindung die im Schritt 2 erhalten wird, zwischen 1 und 20 und vorzugsweise zwischen 5 und 15 % liegt.

16. Verfahren nach Anspruch 1, dadurch gekennzeichnet, daß das molare Verhältnis der im Schritt 2 erhaltenen Verbindung zur Fluorwasserstoffsäure zwischen 5 und 50 und vorzugsweise zwischen 10 und 30 beträgt.

**Claims**

1. Process for the preparation of pentafluoroethoxy- and pentafluoroethylthiobenzene derivatives, which is characterized in that:

- in a first step, there are reacted,
- either a halobenzene with trifluoroethanol in an aprotic solvent in the presence of a strong base and optionally in the presence of a copper salt and/or a complexing agent;
- or a phenolic or thiophenolic derivative with a derivative of general formula (I)

$CF_3$-$CH_2$-O-R' (I)

in which R' denotes a substituent chosen from the group comprising trifluoroacetyl, methanesulphonyl, para-toluenesulphonyl, trichloromethanesulphonyl, chlorosulphonyl, trifluoromethanesulphonyl in the presence of a strong alkaline base, an aprotic solvent and optionally a complexing agent,

- in a second step, the derivative originating from the first step is chlorinated using chlorine gas in the presence of radiations and/or phosphorus trichloride,
- in a third step, the product originating from the second step is fluorinated in anhydrous liquid hydrogen fluoride in the presence of a Levis acid.

2. Process according to Claim 1, characterized in that, during the first step, a bromo- or an iodobenzene is reacted with trifluoroethanolate in the presence of copper bromide or copper chloride in solution in a dipolar aprotic solvent.

3. Process according to Claim 1, characterized in that, during the first step, a halobenzene activated by an electron-withdrawing substituent is reacted with trifluoroethanol in the presence of a base in an aprotic solvent and optionally in the presence of a complexing agent.

4. Process according to Claims 2 or 3, characterized in that the molar ratio of trifluoroethanol to the halo compound is between 0.5 and 5.

5. Process according to Claim 1, characterized in that the compound of formula (I) is chosen from 2,2,2-trifluoroethyl para-toluenesulphonate and 2,2,2-trifluoroethyl trichloromethanesulphonate.

6. Process according to Claim 1, characterized in that the molar ratio of the phenol or the thiophenol to the compound of formula (I) is between 0.5 and 5, and preferably between 0.7 and 1.5.

7. Process according to Claim 1, characterized in that the base is chosen from sodium hydride, potassium hydroxide, sodium hydroxide and sodium.

8. Process according to Claim 1, characterized in that the complexing agent corresponds to the general formula (II)

$N[CHR_1\text{-}CHR_2\text{-}O\text{-}(CHR_3\text{-}CHR_4\text{-}O)_n\text{-}R_5]_3$ (II)

in which formula n is an integer greater than or equal to 0 and smaller than or equal to 10 ($0 \leqslant n \leqslant 10$), $R_1$, $R_2$,

$R_3$ and $R_4$, which are identical or different, denote a hydrogen atom or an alkyl radical containing from 1 to 4 carbon atoms and $R_5$ denotes an alkyl or cycloalkyl radical containing from 1 to 12 carbon atoms, a phenyl radical or a radical of formula:

$C_mH_{2m}C_6H_5$ or $C_mH_{2m+1}C_6H_4$-,

m being between 1 and 12.

9. Process according to Claims 1 to 8, characterized in that the molar ratio of the derivative of formula (II) to the halobenzene, the phenol or the thiophenol is between 0.01 and 0.2.

10. Process according to Claim 1, characterized in that, during the second step, the compounds originating from the first step are reacted with chlorine gas in the presence of radiations.

11. Process according to Claim 10, characterized in that, during the second step, a solvent chosen from carbon tetrachloride or the chlorobenzenes, and preferably carbon tetrachloride, is added.

12. Process according to Claim 10, characterized in that the reaction temperature is between 70 and 200°C.

13. Process according to Claims 11 and 12, characterized in that the reaction temperature is between 70 and 85°C.

14. Process according to Claim 1, characterized in that the Lewis acid is chosen from antimony pentachloride and boron trifluoride.

15. Process according to Claims 1 or 14, characterized in that the molar ratio of the Lewis acid to the compound originating from step 2 is between 1 and 20 %, and preferably between 5 and 15 %.

16. Process according to Claim 1, characterized in that the molar ratio of the compound originating from step 2 to hydrogen fluoride is between 5 and 50 and preferably between 10 and 30.